# EUROPÄISCHE PATENTSCHRIFT

(11) **EP 1 152 778 B1**
(45) Veröffentlichungstag und Bekanntmachung des Hinweises auf die Patenterteilung: **24.09.2003**
(21) Anmeldenummer: 00922483.3
(22) Anmeldetag: 24.02.2000
(51) Int. Cl.: A61L 33/00

(54) **HÄMOKOMPATIBLE OBERFLÄCHEN UND VERFAHREN ZU DEREN HERSTELLUNG**
HEMOCOMPATIBLE SURFACES AND METHOD FOR PRODUCING SAME
SURFACES HEMOCOMPATIBLES ET PROCEDE PERMETTANT DE LES PREPARER

(30) Priorität: 26.02.1999 DE 19908318
(43) Veröffentlichungstag der Anmeldung: 14.11.2001
(73) Patentinhaber: Hoffmann, Michael, 52249 Eschweiler (DE); Horres, Roland, 52223 Stolberg (DE)
(72) Erfinder: Hoffmann, Michael, 52249 Eschweiler (DE); Horres, Roland, 52223 Stolberg (DE)
(74) Vertreter: Bucher, Ralf, Dipl.-Ing. (Univ.)
(86) Internationale Anmeldenummer: EP0001497
(87) Internationale Veröffentlichungsnummer: WO00050106

(56) Entgegenhaltungen:
- WO-A-93/01843
- DE-A- 3 639 561
- US-A- 4 350 629
- US-A- 5 643 712

## Beschreibung

Die vorliegende Erfindung betrifft hämokompatible Oberflächen, die sich dadurch auszeichnen, daß auf- und/oder in die Oberfläche von Werkstoffen Bestandteile der äußeren Schicht von Blutzellen und/oder Mesothelzellen auf- und/oder eingebracht sind. Ferner betrifft die vorliegende Erfindung ein Verfahren zur Herstellung hämokompatibler Oberflächen sowie deren Verwendung in weiten Bereichen des Gesundheitssektors, in der Medizin, Zahnmedizin, Chirurgie, Kosmetik und/oder in Bereichen, die direkt mit Blut, Gewebe und/oder anderen Körperflüssigkeiten in Kontakt stehen.

Die Blutgerinnung stellt sich bei Vertebraten als ein komplexer Prozeß dar, der im Falle einer Verletzung kurzfristig vor lebensbedrohlichen Blutverlusten schützt. Aktiviert wird das Blutgerinnungssystem dabei u.a. durch den Kontakt mit unphysiologischen, also "körperfremden" Substanzen. Substanzen, die das Blutgerinnungssystem aktiv unterdrücken, werden auch als anti-thrombogen bezeichnet. Hingegen werden Substanzen, die das Blutgerinnungssystem gar nicht erst aktivieren als nicht-thrombogen definiert.

Die Aktivierung des Blutgerinnungssytems stellt vor allem bei invasiven Eingriffen ein schwerwiegendes Problem für den Patienten dar. Dies ist insbesondere für diejenigen Menschen der Fall, die auf Implantate, wie beispielsweise Intracoronarstents, Herzklappen, Prothesen, künstliche Gefäßsysteme, Dialysatoren oder Oxygenatoren, Katheter, Biosensoren u.a. angewiesen sind. Auch der Kontakt mit chirurgischen Nahtmaterialien kann zu Problemen führen.
Bislang wird zur Vermeidung der Ausbildung lebensbedrohlicher Gefäßverschlüsse (Thromben) das Blutgerinnungssystem außer Kraft gesetzt oder aktiv unterdrückt. Dies erfolgt in der Regel durch die Gabe von anti-thrombogenen Medikamenten, sogenannten Antikoagulantien. Diese wiederum weisen eine Vielzahl starker Nebenwirkungen für den Patienten auf, wie beispielsweise Thrombozytopenie, Nausea, Erbrechen, Haarausfall, hämorrhagische Hautnekrosen, erhöhte Blutungsneigung etc.. Darüber hinaus ist im Falle der Verwendung von Intracoronarstents oder Herzklappen selbst die vollständige medikamentöse Unterdrückung der Blutgerinnung oftmals kein ausreichender Schutz vor der Ausbildung zum Teil tödlich wirkender Thrombosen.

Für weite Bereiche des Gesundheitssektors, der Medizin, Zahnmedizin, Chirurgie, Kosmetik oder im allgemeinen der Bereiche, die bei invasiven Eingriffen mit Blut und/oder anderen Körperflüssigkeiten in Kontakt stehen, ist daher die Vermeidung der zuvor genannten starken Nebenwirkungen durch Antikoagulantien von großer Bedeutung.

Aus dem Stand der Technik sind verschiedene Verfahren bekannt, die unphysiologische "Fremdoberflächen" durch Beschichtung mit unterschiedlichen Substanzen blut- bzw. gewebeverträglicher (hämokompatibel) machen sollen.

So beschreibt die DE 28 31 360 ein Verfahren zur Beschichtung einer Oberfläche eines medizinischen Gegenstandes mit einer Substanz (Heparin), die das Gerinnungssystem aktiv unterdrückt, also anti-thrombogen ist. Jedoch weist diese Substanz den Nachteil erheblicher Nebenwirkungen für den Patienten auf, wie sie beispielhaft bereits zuvor aufgezählt wurden.

In der DE 44 35 653 werden Materialien mit einer dünnen Lackschicht aus Polymeren, in die zusätzlich Arzneistoffe eingearbeitet sein können, beschichtet, wobei diese Lackschicht im Körper permanent degradiert und somit freigesetzt wird. Nachteile dieser Methode sind zum einen, daß durch den permanenten Zerfall der Beschichtung nur eine zeitlich begrenzte Wirkung möglich ist. Zum anderen ist durch die permanente Ablösung von Lackpartikeln die Gefahr der Ausbildung von Thrombosen, die zu Embolien führen können, sehr hoch.

Die DE 196 30 879 verwendet zur Beschichtung von Substraten ausschließlich chemisch modifizierte Derivate von Polysacchariden. Die Nachteile dieses Verfahrens sind dabei vielgestaltig und reichen von einem übermäßigen präparativen Aufwand über vielstufige Syntheseschritte, einem breiten Spektrum an unerwünschten Nebenreaktionen und mangelhaften Ausbeuten bis hin zu durchweg schlechteren Eigenschaften der Derivate verglichen mit kommerziell erhältlichen anti-thrombogenen Substanzen, wie beispielsweise Heparin.

Verhagen et al. (British Journal of Heamatology, 1996, 95: 542-549) beschreibt die Verwendung von ganzen lebenden Zellen des Endothels bzw. Mesothels zur Besiedlung von Implantaten. Nachteilig an der Verwendung ganzer Zellen ist hier, daß es aufgrund spezifischer Zelloberflächenproteine zu Immunreaktionen kommt, die bei den Patienten zu Abstoßungsreaktionen gegenüber den beschichteten Implantaten führen. Substanzen, die eine solche Immunreaktion auslösen, werden auch als immunogen bezeichnet. Zur Vermeidung einer Abstoßung durch Immunreaktionen muß bei diesem Verfahren ausschließlich Patienteneigenes Zellmaterial benutzt werden. Dies stellt einen weiteren Nachteil dar, da die Anzucht dieser Zellen sehr zeit- und kostenintensiv ist. Problematisch sind bei der Verwendung von ganzen Zellen ferner die hohen Scherkräfte, denen diese Zellen im Blutstrom ausgesetzt sind. Dies führt zu verstärkter Degradation der Zellen an den Oberflächen, was sich negativ auf die Haltbarkeit der beschichteten Implantate auswirkt.

Auch die WO 93/01843, WO 95/29712 und DE 195 05 070 beschreiben die Verwendung von ganzen lebenden Endothelzellen zur Beschichtung von unphysiologischen Materialien bzw. die Verwendung von Substanzen, die ein Anwachsen von lebenden Endothelzellen auf künstlichen Materialien begünstigen. Jedoch liegt auch hier allen Verfahren die Kultivierung lebender Endothelzellen zugrunde, verbunden mit den zuvor bereits beschriebenen Nachteilen hinsichtlich des Zeit- und Kostenaufwandes bzw. der erheblichen Einschränkung, daß das beschichtete Material nicht universell eingesetzt werden kann, sondern für jeden einzelnen Patienten gesondert hergestellt werden muß.

Aus der Patentschrift DE 36 39 561 ist die Herstellung von Substraten, die mit dem spezifischen Endothelzelloberflächen-Proteopolysaccharid HS-I beschichtet werden, bekannt. Nachteile dieses Verfahrens sind, daß auch hier zur Isolierung dieser Komponenten größere Mengen an Patienteneigenen Endothelzellen benötigt werden. Dies erfordert für jeden einzelnen Patienten eine zeit- und kostenintensive Kultivierung seiner körpereigenen Endothelzellen, an die sich zusätzlich eine aufwendige Präparation des Proteopolysaccharid HS-I anschließt. Aufgrund dessen ist eine großtechnische Herstellung von HS-I und somit eine wirtschaftliche Nutzung dieses Verfahrens zur Beschichtung von Implantaten nicht realisierbar.

US-A-4 350 629 beschreibt hämokompatible Oberflächen welche Glucosaminoglykan beliebigen Ursprungs enthalten.

Die hier vorliegende Erfindung hat sich demgemäß die Aufgabe gestellt, blut- bzw. gewebeverträgliche, also hämokompatible Oberflächen zur Verfügung zu stellen, die die zuvor genannten Nachteile nicht aufweisen und gleichzeitig für eine Herstellung im großtechnischen Maßstab geeignet sind.

Diese Aufgabe wird erfindungsgemäß durch hämokompatible Oberflächen gelöst, die sich dadurch auszeichnen, daß sie als Werkstoffe künstliche und/oder natürliche organische und/oder anorganische Verbindungen und/oder Mischungen davon und/oder Materialien, die bei invasiven Eingriffen mit Blut und/oder anderen Körperflüssigkeiten in Kontakt kommen und/oder tierische Organe und/oder Organteile enthalten und auf und/oder in die Oberfläche dieser Werkstoffe Oligosaccharid-Polysaccharid- und/oder Lipid-Anteile der Glykoproteine, Glykolipide und/oder Proteoglykane aus der äußeren Schicht von Blutzellen und/oder Mesothelzellen auf- und/oder eingebracht sind.

Die erfindungsgemäß hämokompatiblen Oberflächen ahmen somit im wesentlichen die äußere Oberfläche von Blut- und/oder Mesothelzellen nach, gleichbedeutend mit der Imitation der natürlichen Oberfläche nichtthromogener Zellen und/oder Gewebe.
Das Blutgerinnungssystem wird folglich durch die hämokompatiblen Oberflächen weder aktiviert noch aktiv unterdrückt. Folglich kann eine Blutgerinnung, die z.B. durch sekundäre Verletzungen (Schnittwunden o.ä.) ausgelöst wird, vollkommen natürlich und ungestört ablaufen.
Ein weiterer Vorteil der vorliegenden Erfindung ist, daß eine Anhaftung von Zellen, wie beispielsweise Thrombocyten auf den erfindungsgemäß hämokompatiblen Oberflächen ausbleibt. Dies ist erfindungsgemäß erwünscht, da dadurch das Risiko der Ausbildung von Thromben, d.h. die Gefahr einer Thrombose (Embolie) für den behandelten Patienten minimiert ist. Die erfindungsgemäß hämokompatiblen Oberflächen sind frei von Nebenwirkungen.

Erfindungsgemäß zeichnen sich die hämokompatiblen Oberflächen ferner dadurch aus, daß sie langfristig nicht-thrombogen sind. D.h. ihre vorteilhaften Eigenschaften verbrauchen sich nicht im Laufe der Zeit, so wie es beispielsweise bei pharmazeutisch aktiven Systemen (z.B. Release-System) der Fall ist. Aufgrund dessen eignen sich die erfindungsgemäßen Oberflächen auch zum Dauereinsatz, so daß zusätzliche Belastungen und Risiken für die Patienten durch wiederholte invasive Eingriffe zur Erneuerung der Implantate minimiert werden.

Erfindungsgemäß enthalten die hämokompatiblen Oberflächen als Werkstoffe künstliche und/oder natürliche organische und/oder anorganische Verbindungen und/oder Mischungen davon und/oder Materialien, die bei invasiven Eingriffen mit Blut und/oder anderen Körperflüssigkeiten in Kontakt kommen und/oder tierische Organe und/oder Organteile auf und/oder in deren Oberfläche Bestandteile der äußeren Schicht von Blutzellen und/oder Mesothelzellen auf- und/oder eingebracht sind.

Unter Werkstoffen sind im Sinne der Erfindung sämtliche Materialien zu verstehen, die sich erfindungsgemäß dazu eignen mit Zellbestandteilen beaufschlagt zu werden. Ebenso zählen hierzu alle Materialien, die bei invasiven Eingriffen oder im Zuge einer entsprechenden Nachsorge mit Blut und/oder Körperflüssigkeiten in Kontakt kommen können.

Unter organischen Verbindungen sind beispielsweise synthetisch hergestellte oder natürlich vorkommende hochmolekulare Stoffe und deren Derivate zu verstehen. Beispiele hierfür sind u.a. alle Formen von Kunststoffen, Elastomeren, Silikonen oder Faserstoffen. Hierzu zählen z.B. Polyethylene (PE), Polyvinylchloride (PVC), Polyurethane (PUR), Polyamide (PA), Phenoplaste (PF), Aminoplaste, Polystyrol, Polyester, Harze, Silikone, Kautschuke, Chemiefaserstoffe, Zellulosefaserstoffe, Zellulosemembranen, Proteinfaserstoffe, Collagene sowie Derivate davon oder Kombinationen davon. Ferner sind auch Mischungen dieser Polymere, sogenannte Polymerblends erfindungsgemäß umfaßt.

In einer besonderen Ausführungsvariante der vorliegenden Erfindung können die erfindungsgemäß hämokompatiblen Oberflächen als Werkstoffe tierische Organe, Organteile oder Gefäßsysteme enthalten. Dies können beispielsweise Herzklappen und/oder Gefäßsysteme sein, wobei sich als Quelle Schweine oder Rinder besonders eignen.

Als Beispiele für anorganische Verbindungen enthalten die erfindungsgemäß hämokompatiblen Oberflächen Metalle, Metalloxide, Legierungen, oder Keramiken, Gläser und/oder Mineralien sowie Derivate davon oder alle denkbaren Kombinationen und/oder Mischungen davon.
Erfindungsgemäß sind alle Kombinationsmöglichkeiten von Werkstoffen denkbar. Die Beispiele führen die vorliegende Erfindung näher aus, sind jedoch nicht limitierend.

Erfindungsgemäß sind in und/oder auf der Oberfläche der Werkstoffe Bestandteile der äußeren Schicht von Blutzellen und/oder Mesothelzellen ein- und/oder aufgebracht.

In einer Ausführungsform der vorliegenden Erfindung enthalten die hämokompatiblen Oberflächen in und/oder auf der Oberfläche von Werkstoffen Glykoproteine, bevorzugt Glykophorine. Diese Glykophorine zeichnen sich u.a. durch nicht-thrombogene Eigenschaften aus und eignen sich dadurch hervorragend zur Herstellung erfindungsgemäß hämokompatibler Oberflächen.
Durch die Glykophorine der äußeren Schicht von Erythrocyten ist u.a. die Blutgruppenzugehörigkeit eines Menschen festgelegt. Analog zu den verschiedenen Blutgruppen A, B, AB und 0 enthalten die korrespondierenden Erythrocyten Glykophorin A, Glykophorin B oder Glykophorin 0 oder entsprechende Mischungen davon.
Eine mögliche Immunantwort durch Kreuzreaktionen nicht miteinander verträglicher Blutgruppen, d.h. ein Verklumpen von Blut (Koagulation) kann hier auf einfache Weise dadurch ausgeschlossen werden, daß vor einem invasiven Eingriff eine Abstimmung erfolgt, hinsichtlich der Blutgruppe des zu behandelnden Patienten und der auf und/oder in die Oberfläche von Werkstoffen auf- und/oder eingebrachten Glykophorine der zur Applikation beabsichtigten erfindungsgemäß hämokompatiblen Oberflächen. Entsprechende Bluttests sind gängige Laborpraxis und werden entsprechend routinemäßig durchgeführt. Unter der Voraussetzung der Beachtung der Blutgruppenverträglichkeit sind somit auch Glykophorin enthaltende hämokompatible Oberflächen universell einsetzbar, d.h. nicht an einen einzigen Patienten gebunden.

Die vorliegende Erfindung betrifft hämokompatible Oberflächen enthaltend auf und/oder in der Oberfläche der Werkstoffe Oligosaccharid-, Polysaccharid- und/oder Lipid-Anteile der Glykoproteine, Glykolipide und/oder Proteoglykane aus der äußeren Schicht von Blutzellen und/oder Mesothelzellen.

In einer weiteren Ausführungsvariante der vorliegenden Erfindung enthalten die hämokompatiblen Oberflächen auf und/oder in der Oberfläche der Werkstoffe Glykosphingolipide.

Ferner können die hämokompatiblen Oberflächen der vorliegenden Erfindung als Oligosaccharid- oder Polysaccharid-Anteile der Proteoglykane Hyaluronsäuren, Chondroitinsulfate, Dermatansulfate, Heparansulfate, Keratansulfate oder Mischungen davon enthalten. In einer bevorzugten Ausführungsform der vorliegenden Erfindung enthalten die hämokompatiblen Oberflächen Heparansulfat der Erythrocyten-Plasmamembran tierischer und/oder menschlicher Herkunft.

Die erfindungsgemäß hämokompatiblen Oberflächen sind frei von Nebenwirkungen, wie sie z.B. durch chemisch oder pharmazeutisch aktive Beschichtungen bekannt sind.

Bei den zuvor genannten Bestandteilen der Blut- und/oder Mesothelzellen handelt es sich um nicht-immunogene Zellbestandteile. Folglich zeichnen sich die erfindungsgemäß hämokompatiblen Oberflächen dadurch aus, daß sie außerdem nicht-immunogen sind. Das heißt, sie lösen bei den Patienten keine Immunreaktion aus, wodurch die Gefahr einer Abstoßung der hämokompatiblen Oberflächen minimiert ist.

Erfindungsgemäß sind die hämokompatiblen Oberfächen nicht-thrombogen und/oder nicht-immunogen.

Ein weiterer Vorteil ist, daß aufgrund der erfindungsgemäßen festen Verankerung der nicht-thrombogenen Bestandteile der äußeren Schicht der Blut und/oder Mesothelzellen auf den Werkstoffen nahezu keine Degradation an den hämokompatiblen Oberflächen stattfindet. Eine Gefahr der Bildung von Embolien durch Thrombosen ist somit minimiert. Ferner erfolgt keine Anlagerung von Zellen, wie z.B. Thrombocyten auf den erfindungsgemäß hämokompatiblen Oberflächen. Dies minimiert ebenfalls die Gefahr von Thrombosen.

Gegenstand der vorliegenden Erfindung ist ferner ein Verfahren zur Herstellung der erfindungsgemäß hämokompatiblen Oberflächen, bei dem Glykophorine, Oligosaccharid-, Polysaccharid- und/oder Lipid-Anteile der Glykoproteine, Glykolipide und/oder Proteoglykane aus der äußeren Schicht von Blutzellen und/oder Mesothelzellen isoliert werden und diese Zellbestandteile durch physikalische oder chemische Bindung auf und/oder in die Oberfläche von Werkstoffen aus künstlichen und/oder natürlichen organischen und/oder anorganischen Verbindungen und/oder Mischungen davon und/oder Materialien, die bei invasiven Eingriffen mit Blut und/oder anderen Körperflüssigkeiten in Kontakt kommen und/oder tierischen Organen und/oder Organteilen auf- und/oder eingebracht werden.

Erfindungsgemäß werden die Bestandteile der äußeren Schicht von Blutzellen aus Vollblut und/oder aus daraus gewonnenen Zellfraktionen menschlicher oder tierischer Herkunft isoliert. D.h., daß die Zellbestandteile aus Erythrocyten, Leukocyten und/oder Thrombocyten oder Gemischen davon isoliert werden. Bevorzugt werden Gemische aus Erythrocyten und Leukocyten. Besonders bevorzugt werden Erythrocyten.

Die Bestandteile aus der äußeren Schicht von Mesothelzellen werden erfindungsgemäß aus Omentum, Peritoneum und/oder inneren Organen isoliert

Eine preiswerte und leicht zugängliche Quelle für diese Ausgangsmaterialien können beispielsweise Schlachtabfälle sein.

Die Isolierung der Bestandteile der äußeren Schicht der Blutzellen, Mesothelzellen oder des mesothelzellreichen Gewebes erfolgt dabei in an sich bekannter Weise. Beispielsweise sind hier folgende Verfahren oder deren Kombinationen denkbar: Zerkleinerung, Extraktion, Filtration, Fällung, Gelfiltration, Ionenaustauschchromatographie, Affinitätschromatographie, Elektrophorese, enzymatische oder chemische Abbauten, Trocknung, Auflösung, Dialyse, Ultrafiltration etc..

Erfindungsgemäß wird zur Auf- und/oder Einbringung der Zellbestandteile auf und/oder in die Oberfläche der Werkstoffe eine chemische Immobilisierung, Photoimmobilisierung, Adhäsion, Trocknung oder eine Kombination davon durchgeführt. Dabei können kovalente, ionische, nebenvalente bzw. elektrostatische oder adhäsive Bindungen oder Kombinationen davon zwischen den Bestandteilen der äußeren Schicht der Zellen und den Oberflächen der Werkstoffe ausgebildet werden. Bevorzugt erfolgt die Auf- bzw. Einbringung der Bestandteile der äußeren Zellschicht auf/in die Oberfläche von Werkstoffen durch kovalente Bindungen.

Ein besonderer Vorteil der vorliegenden Erfindung ist, daß das erfindungsgemäße Herstellungsverfahren enorme wirtschaftliche Verbesserungen gegenüber bisher bekannten Verfahren auf sich vereint und folglich die erfindungsgemäß hämokompatiblen Oberflächen zur Herstellung im großtechnischen Maßstab geeignet sind.
Dies basiert beispielsweise darauf, daß erfindungsgemäß Zellbestandteile und keine lebenden Zellen eingesetzt werden, daß keine körpereigenen (Endothel-) Zellen des Patienten verwendet werden müssen, daß das Ausgangsmaterial zur Isolierung dieser Zellbestandteile preiswert und in großen Mengen verfügbar ist (Schlachtabfälle), daß somit keine Zellkultivierung erforderlich ist, die sehr zeit- und kostenintensiv ist. Ein Vorteil der erfindungsgemäß hämokompatiblen Oberflächen ist femer, daß sie universell einsetzbar sind und nicht nur an den Einsatz für einen einzigen Patienten gebunden sind. Dies stellt vor allem bei Notoperationen einen lebenswichtigen Vorteil für die Patienten dar.

Die Anwendungsgebiete der vorliegenden Erfindung sind weit gestreut. Die vorliegende Erfindung betrifft die Verwendung hämokompatibler Oberflächen in weiten Bereichen des Gesundheitssektors, in der Medizin, Zahnmedizin, Chirurgie oder Kosmetik und/oder in Bereichen, die bei invasiven Eingriffen mit Blut, Gewebe und/oder anderen Körperflüssigkeiten in Verbindung kommen.

Im folgenden wird die Erfindung unter Bezugnahme auf die Beispiele näher erläutert, die jedoch nicht limitierend sind:

### 1.) Isolierung von Erytrocytenplasmamembran Heparansulfat:

Ein Liter serumfrei gewaschene Erythrocyten werden in 1 Liter 0,154 molarem Phosphatpuffer pH 7 suspendiert und mit 1 U/ml Papain versetzt. Nach 2 Stunden Inkubation bei 56°C wird 20 Minuten bei 3000 g abzentrifugiert und der Überstand anschließend dekantiert. In diesem Überstand werden 100 ml DEAE-Sepharose CL-6B lonenaustauscher-Gel der Firma Pharmacia Biotech suspendiert. Das so beladene Gel wird noch dreimal in 0,1 molarer Kochsalz-Lösung gewaschen und in eine Chromatographiesäule gefüllt. Die Elution erfolgt mittels eines linearen Kochsalzgradienten im Bereich von 0,1 bis 0,8 mol/l über einem Gesamt-Elutionsvolumen von 2 Litern. Es werden 200 Fraktionen zu je 10 ml Volumen gesammelt. Die Fraktionen, die mit Dimethylmethylenblau (DMMB) der Firma Fluka nach der Methode beschrieben bei Chandrasekhar et al (Analytical Biochemistry, 161 (1987): 103-108) eine positive Farbreaktion ergeben, werden vereinigt. Die Lösung der gesammelten Fraktionen wird bei 26,7 hPa (20 Torr)und 40°C eingeengt und gegen Wasser dialysiert. Das Dialysat wird auf ein Volumen von 100 ml und eine Konzentration von 0,03 mol/l Natriumacetat, 0,073 mol/l Tris (Tris(hydroxymethyl)aminomethan der Firma Fluka) und pH 8.0 eingestellt, mit 1 U Chondroitinase ABC versetzt und 15 Stunden bei 37°C inkubiert. Nach Dialysieren gegen Wasser und Einengen unter Wasserstrahlvakuum wird die erhaltene Lösung erneut auf eine Säule mit 100 ml DEAE-Sepharose CL-6B der Firma Pharmacia Biotech. aufgetragen und wie zuvor beschrieben eluiert. Die DMMB-positiven Gradientenfraktionen werden dialysiert, unter Wasserstrahlvakuum auf ein Volumen von 1 ml eingeengt und auf einer Säule zur präparativen Gelfiltration (60 cm x 2 cm) unter Verwendung eines Sephacryl S-300 Gels (Pharmacia Biotech.) chromatographiert. Es werden 60 Fraktionen zu je 2 ml Volumen gesammelt, mit DMMB detektiert und die positiven Fraktionen vereinigt. Nach wiederholter Dialyse und Lyophilisation erhält man das aufgereinigte Erythrocytenplasmamembran-Heparansulfat.

### 2.) Isolierung von Leukocytenoberflächen-Proteochondroitinsulfat:

Ein Liter Citratblut wird 10 min in einer Zentrifuge mit Ausschwingrotor bei 3000 g zentrifugiert und das überstehende Plasma abgesaugt. Das Zellsediment wird mit 2 Litern auf 4°C gekühlter 1%iger Ammoniumoxalatlösung gemischt und 30 min bei derselben Temperatur inkubiert. Nach 5 minütiger Zentrifugation bei 500 g wird der rote Überstand verworfen und das Pellet in 2 Litern auf 4°C gekühlter 1%iger Ammoniumoxalatlösung suspendiert, 5 min bei 500 g zentrifugiert und der Waschvorgang, wie oben beschrieben noch zweimal wiederholt. Der nun farblose Überstand wird verworfen und das gewaschene Zellsediment (Ausbeute: 12 x 10⁷ - 10 x 10⁹ Zellen in 2 Litern Triton X-100 Puffer (0,5% Triton X-100, 10 mM Tris-HCI, 150 mM NaC1, pH 8) unter ständigem Rühren bei 25°C über 2 Stunden lysiert. Der Detergenzextrakt wird 60 min bei 10.000 g zentrifugiert, dekantiert und im Überstand werden im 10 ml DEAE Sephadex A50 lonenaustauscher Gel der Firma Pharmacia Biotech. suspendiert und sedimentiert. Das so beladene Gel wird noch dreimal in 0,1 molarer Kochsalz-Lösung gewaschen und in eine Chromatographiesäule gefüllt. Mit einem linearen Kochsalzgradienten von 0,1 bis 0,8 mol/l über ein Gesamt-Elutionvolumen von 2 Litern wird die Säule eluiert. Es werden 100 Fraktionen zu je 2 ml Volumen gesammelt und die Fraktionen, die mit Dimethylmethylenblau (DMMB der Firma Fluka) eine positive Farbreaktion ergeben, vereinigt. Die Lösung wird bei 26,7 hPa (20 Torr) und 40°C eingeengt und gegen Wasser dialysiert. Das Dialysat wird auf ein Volumen von 100 ml und eine Konzentration von 0,1 mmol/l Calciumacetat und 0,1 mol/l Natriumacetat eingestellt, mit Essigsäure auf pH 7 titriert, mit je 1 U Heparinase I, Heparinase II und Heparinase III versetzt und 15 Stunden bei 37°C inkubiert.
Nach Dialysieren gegen Wasser und Einengen unter Wasserstrahlvakuum wird die resultierende Lösung erneut auf eine Säule mit 10 ml DEAE Sephadex A50 der Firma Pharmacia Biotech. aufgetragen und wie oben beschrieben eluiert. Die DMMB-positiven Gradientenfraktionen werden dialysiert, unter Wasserstrahlvakuum auf ein Volumen von 1 ml eingeengt und auf einer Säule zur präparativen Gelfiltration (60 cm x 2 cm) unter Verwendung eines Sepharose Cl-4B Gels der Firma Pharmacia Biotech. chromatographiert. Es werden 60 Fraktionen zu je 2 ml Volumen gesammelt, mit DMMB detektiert und die positiven Fraktionen vereinigt. Nach wiederholter Dialyse und Lyophilisation erhält man das aufgereinigte Leukocytenoberflächen-Proteochondroitinsulfat.

### 3.) Isolierung von Heparansulfat/Chondroitinsulfat-Gemisch aus Omentum:

Ein Kilogramm frisches Rinderomentum wird mit 0,9%iger NaCI-Lösung gewaschen, gefriergetrocknet, gemahlen und mit 1 Liter Aceton durch Rühren über Nacht bei Raumtemperatur entfettet. Nach dem Abfiltrieren und Trocknen wird das resultierende Pulver in 6 molarer Harnstoff-Lösung suspendiert und über Nacht bei Raumtemperatur gerührt. Nach Zentrifugation bei 3000 g für 1 Stunde wird der schleimige Überstand dekantiert, auf 4°C gekühlt, mit dem gleichen Volumen 4°C kalter 1 molarer NaOH gemischt und 15 Stunden bei 4°C inkubiert. Danach wird mit verdünnter HCI neutralisiert, gegen Wasser dialysiert, 1 Stunde bei 3000 g zentrifugiert und der Überstand dekantiert. Im Überstand werden 100 ml DEAE-Sepharose CL-6B Ionenaustauscher-Gel der Firma Pharmacia Biotech. suspendiert und sedimentiert. Das so beladene Gel wird noch dreimal in 0,1 molarer Kochsalz-Lösung gewaschen und in eine Chromalographiesäule gefüllt. Mit einem linearen Kochsalzgradienten von 0,1 bis 0,8 mol/l über einem Gesamt-Elutionsvolumen von 2 Litern wird die Säule eluiert. Es werden 200 Fraktionen zu je 10 ml Volumen gesammelt und die Fraktionen, die mit Dimethylmethylenblau (DMMB) eine positive Farbreaktion ergeben, vereinigt. Die Lösung wird bei 26,7 hPa (20 Torr) und 40°C eingeengt und gegen Wasser dialysiert. Unter Wasserstrahlvakuum wird erneut auf ein Volumen von 5 ml eingeengt und auf einer Säule zur präparativen Gelfiltration (60 cm x 5 cm) unter Verwendung eines Sephacryl S-300 Gels der Firma Pharmacia Biotech. chromatographlert. Es werden 60 Fraktionen zu je 10 ml Volumen gesammelt, mit DMMB detektiert und die positiven Fraktionen vereinigt. Nach wiederholter Dialyse und Lyophilisation erhält man das aufgereinigte Mesothelzelloberflächen-Glykosaminoglykan-Gemisch.

### 5.) Immobilisierung von Mesothelzelloberflächen Chondroitinsulfat mit (N-Cyclohexyl-N'-2- morpholinoethyl)carbodiimidmethyl p-Toluolsulfonat (CME-CDI) auf funktionalisierte Zelluloseoberflächen:

100 mg Zellulosemembran werden in eine 2 prozentige Lösung von 3-Aminopropyl-triethoxysilan in Ethanol/Wasser (50:50) gegeben und 24 Stunden bei 45°C gerührt. Danach werden die Membranen mit viel Wasser gewaschen und getrocknet. Die so behandelten Membranen werden in eine Lösung von 1 mg Mesothelzelloberflächen Chondroitinsulfat in 80 ml 0,1 molarer 2-(N-Morpholino)ethansulfonsäure-Puffer pH 4,75 getaucht. Über einen Zeitraum von 6 Stunden werden bei 4°C 200 mg (N-Cyclohexyl-N'-2-morpholinoethyl)carbodiimidmethyl p-Toluolsulfonat (CME-CDI) der Firma Sigma in 10 mg Portionen zugegeben und über Nacht bei 4°C weitergerührt. Danach wird 2 Stunden in 4 molarer NaCl-Lösung gerührt, mit viel Wasser gespült und an der Luft getrocknet.

### 6.) CNCI Immobilisierung von Sphingoglycolipid auf Glas:

Ein Glas, beispielsweise ein Deckgläschen für die Mikroskopie, wird 6 Stunden in 5 ml Chromschwefelsäure gerührt. Dann wird mit viel Wasser gewaschen, luftgetrocknet und in 15 ml Dioxan auf 50°C erhitzt. Anschließend werden 2,5 ml einer 2 molaren N,N'-Diisopropylethylamin-Lösung in Dioxan zugegeben und 30 min gerührt. Dann werden 2,5 ml einer 1 molaren CNCI-Lösung in Dioxan zugeführt und für weitere 2 Stunden gerührt. Anschließend wird zuerst mit Dioxan gewaschen, dann mit Dioxan/Wasser und schließlich mit reinem Wasser gewaschen. Das so modifizierte Glas wird in 20 ml einer Lösung aus 1 mol/l Ethylendiamin und 0,1 mol/l NaHCO₃ gegeben, dann auf 50°C erwärmt und 72 Stunden bei dieser Temperatur gerührt. Anschließend werden 0,1 mg Sphingoglycolipid aus humanen Erythrocyten in 20 ml 0,1 molarer NaHCO₃ gelöst und zusammen mit dem substituierten Glas 110 Stunden bei 60°C gerührt. Dann werden 2,5 ml Ethanolamin hinzu gegeben und weitere 30 Minuten gerührt. Das beschichtete Glas wird mit 4 molarer NaCl-Lösung und anschließend mit viel Wasser gewaschen und an der Luft getrocknet.

### 7.) Immobilisierung von Erythrocytenplasmamembran-Heparansulfat auf die Oxidschicht von Nickel, Titan, Aluminium oder ähnlichen Metallen:

Das Metallwerkstück wird vier Stunden im Ultraschallbad mit heißem Wasser gereinigt, mit Aceton gewaschen und eine Stunde in einem Soxhlet-Extraktor mit Chloroform entfettet. Das so gereinigte Werkstück wird getrocknet und 2-15 min unter Rühren in eine 0,01 -0,1 molare Lösung von ω-Hexadecenyltrichlorsilan in Bicyclohexyl getaucht, zweimal mit Chloroform und Wasser gewaschen und 15 min im Soxhlet-Extraktor mit Chloroform extrahiert. Das Werkstück wird bei O°C 45 min in eine Lösung von 2 ml Aceton und 100 mg KmnO₄ in 18 ml Wasser getaucht und durch die einen CO₂-Strom geleitet. Danach wird es für 15 Sekunden in eine 20%ige Lösung von Natriumbisulfit in Wasser getaucht, mit Wasser gewaschen und getrocknet.
Das Werkstück wird über Nacht in einer Lösung von 29,25 g Paratoluylsulfonylchlorid in 900 ml Aceton und 180 ml Pyridin bei 40°C gerührt. Anschließend wird das Werkstück mit Wasser und Methanol gewaschen und 40 Stunden lang bei 60°C in einer Lösung von 1 mmol/l Diaminododekan in 1 Liter Dimethylformamid gerührt. Danach wird das Werkstück sukzessive mit Wasser, 1 mol/l Sodalösung, 1 mmol/l Salzsäure und Wasser gewaschen. Das so vorbereitete Werkstück wird für 90 Minuten in einer Borat-Pufferlösung (Natriumtetraborat 0,065 mol/l, pH 9,5) gerührt. Schließlich wird in einer Lösung aus 0,3 g 4-Azido-1-Fluoro-2-Nitrobenzol in einem Liter Ethanol über Nacht bei 37°C gerührt. 0,5 g Erythrocytenplasmamembran-Heparansulfat werden in einem Liter 0,1 molaren 2-(N-Morpholino)ethansulfonsäure-(MES)-Puffer pH 4,75 gelöst und mit dem Werkstück bei 4°C für 48 Stunden gerührt. Das Erythrocytenplasmamembran-Heparansulfat wird kovalent immobilisiert 10 minütige Belichtung mit einer Hochdruck-Quecksilberlampe. Nach Waschen mit 4 molarer Kochsalzlösung für 40 Minuten wird das Werkstück mit Wasser gewaschen und anschließend getrocknet.

### 8.) Photochemische Immobilisierung von Leukocytenplasmamembran-Chondroitinsulfat auf Zellulose:

3 g Zellulosemembran werden in 4 molarer NaOH 2 Stunden quellen gelassen, dreimal mit Wasser gewaschen, einmal mit Wasser/Aceton und einmal mit Aceton gewaschen. Die so aktivierte Zellulose wird über Nacht in einer Lösung von 29,25 g Paratoluylsulfonylchlorid in 900 ml Aceton und 180 ml Pyridin bei 40°C gerührt. Anschließend wird die Zellulosemembran mit Wasser und Methanol gewaschen. Die resultierende veresterte Zellulosemembran wird nun über 40 Stunden bei 60°C in einer Lösung von 1 mmol/l Diaminododekan in 1 Liter Dimethylformamid gerührt. Danach wird die Membran sukzessive mit Wasser, 1 mol/l Sodalösung, 1 mmol/l Salzsäure und Wasser gewaschen. Die so erhaltene Aminozellulose wird für 90 Minuten in einer Borat-Pufferlösung (Natriumtetraborat 0,065 molar, pH 9,5) gerührt. Schließlich wird die Membran in einer Lösung aus 0,3 g 4-Azido-1-Fluoro-2-Nitrobenzol in einem Liter Ethanol aber Nacht bei 37°C gerührt. 0,5 g Leukocytenoberflächen-Chondroitinsulfat werden in einem Liter 0,1 molaren 2-(N-Morpholino)ethansulfonsäure-Puffer pH 4,75 gelöst und mit 2,5 g der wie oben beschrieben hergestellten Azido-Zellulose bei 4°C für 48 Stunden gerührt. Das Leukocytenoberflächen-Chondroitinsulfat wird kovalent immobilisiert durch 10 minütige Belichtung mit einer Hochdruck-Quecksilberlampe. Nach Waschen mit 4 molarer Kochsalzlösung für 40 Minuten und Wasser wird die Zellulosemembran getrocknet.

### 9.) Immobilisierung von Glycophorin A mit Glutardialdehyd auf Silikon:

1 g Silikonfolie wird mit 20 ml Wasser und 2 ml 3-Aminopropyltriethoxysilan versetzt und der pH-Wert auf 3,5 eingestellt. Dann wird für 2 Stunden auf 75°C erhitzt, mit Wasser gewaschen und getrocknet. Das so erhaltene Aminogruppen-haltige Silikon wird mit einer 2,5 prozentigen Lösung von Glutardialdehyd in 0,05 molarem Natriumphosphatpuffer versetzt und auf pH 7 eingestellt. Nach 60 Minuten Rühren bei Raumtemperatur wird das so hergestellte aktivierte Silikon mit einer 0,1%igen Lösung von Glycophorin A (Sigma) unter Rühren 2-4 Stunden umgesetzt und mit Wasser gewaschen.

### 10.) Immobilisierung von Erythrocytenplasmamembran-Heparansulfat auf Polyvinylchlorid (PVC):

0,5 g Eisen-II-sulfat, 100 µl konzentrierte Schwefelsäure und 2 ml Methacrvlsäure werden in 250 ml Wasser gelöst. Zu dieser Lösung werden 125 mg Natriumdisulfit sowie 125 mg Kaliumperoxodisulfat zugegeben. Anschließend wird diese Lösung zwei Stunden bei Raumtemperatur durch einen ringförmigen 1 m langen PVC-Schlauch von 3 mm Innendurchmesser gepumpt. Die dabei ablaufende Pfropfpolymerisation wird durch Zugabe von 100 mg Hvdrochinon abgebrochen. Dann wird der Schlauch gründlich mit Wasser gewaschen. Eine auf 4°C gekühlte Lösung von 250 mg CME-CDI (N-Cyclohexyl-N'-2-morpholinoethyl)carbodiimidmethyl p-Toluolsulfonat in 250 ml 0,1 molarem 2-(N-Morpholino)ethansulfonsäure-Puffer pH 4,75 wird bei 4°C 30 min im Kreis durch den Schlauch gepumpt. Der so aktivierte Schlauch wird mit 0,1 molarem 2-(N-Morpholino)ethansulfonsäure-Puffer pH 4,75 gewaschen. Dann wird eine Lösung von 1 mg Erythrocytenplasmamembran-Heparansulfat in 0,1 molarem 2-(N-Morpholino)ethansulfonsäure-Puffer pH 4,75 bei 4°C 15 Stunden lang im Kreis durch den Schlauch gepumpt. Abschließend wird der Schlauch mit 4 molarer Kochsalzlösung und dann mit Wasser gespült.

## Patentansprüche

1. Hämokompatible Oberflächen, **dadurch gekennzeichnet, daß** sie als Werkstoffe künstliche und/oder natürliche organische und/oder anorganische Verbindungen und/oder Mischungen davon und/oder Materialien, die bei invasiven Eingriffen mit Blut und/oder anderen Körperflüssigkeiten in Kontakt kommen und/oder tierische Organe und/oder Organteile enthalten und auf und/oder in die Oberfläche dieser Werkstoffe Oligosaccharid-, Polysaccharid- und/oder Lipid-Anteile der Glykoproteine, Glykolipide und/oder Proteoglykane aus der äußeren Schicht von Blutzellen und/oder Mesothelzellen auf- und/oder eingebracht sind.

2. Hämokompatible Oberflächen nach Anspruch 1, **dadurch gekennzeichnet, daß** sie nicht-thrombogen und/oder nicht-immunogen sind.

3. Hämokompatible Oberflächen nach einem der Ansprüche 1 oder 2 enthaltend auf und/oder in der Oberfläche der Werkstoffe Glykophorine.

4. Hämokompatible Oberflächen nach einem der Ansprüche 1 - 3 enthaltend auf und/oder in der Oberfläche der Werkstoffe Glykosphingolipide.

5. Hämokompatible Oberflächen nach einem der Ansprüche 1 - 4 enthaltend auf und/oder in der Oberfläche der Werkstoffe als Oligosaccharid- und/oder Polysaccharid-Anteile der Proteoglykane Hyaluronsäuren, Chondroitinsulfate, Dermatansulfate, Heparansulfate, Keratansulfate oder Mischungen davon.

6. Hämokompatible Oberflächen nach einem der Ansprüche 1 - 5 enthaltend auf und/oder in der Oberfläche der Werkstoffe Heparansulfat der Erythrocyten-Plasmamembran tierischer und/oder menschlicher Herkunft.

7. Hämokompatible Oberflächen nach einem der Ansprüche 1 - 6 enthaltend als Werkstoffe hochmolekulare organische Verbindungen und/oder Metalle, Metalloxide, Legierungen, Keramiken, Gläser, Mineralien und/oder Mischungen der zuvor genannten Werkstoffe.

8. Verfahren zur Herstellung hämokompatibler Oberflächen, **dadurch gekennzeichnet, daß**
a) Glykophorine und/oder Oligosaccharid-, Polysaccharid- und/oder Lipid-Anteile der Glykoproteine, Glykolipide und/oder Proteoglykane aus der äußeren Schicht von Blutzellen und/oder Mesothelzellen isoliert werden und
b) diese Zellbestandteile durch physikalische oder chemische Bindung auf und/oder in die Oberfläche von Werkstoffen aus künstlichen und/oder natürlichen organischen und/oder anorganischen Verbindungen und/oder Mischungen davon und/oder Materialien, die bei invasiven Eingriffen mit Blut und/oder anderen Körperflüssigkeiten in Kontakt kommen und/oder tierischen Organen und/oder Organteilen auf- und/oder eingebracht werden.

9. Verfahren nach Anspruch 8, **dadurch gekennzeichnet, daß** die Bestandteile der äußeren Schicht von Blutzellen aus Vollblut und/oder aus daraus gewonnenen Zellfraktionen menschlicher oder tierischer Herkunft isoliert werden.

10. Verfahren nach einem der Ansprüche 8 oder 9, **dadurch gekennzeichnet, daß** Zellbestandteile aus Erythrocyten, Leukocyten und/oder Thrombocyten und/oder Gemischen davon isoliert werden.

11. Verfahren nach einem der Ansprüche 8 - 10, **dadurch gekennzeichnet, daß** Bestandteile der äußeren Schicht von Mesothelzellen aus Omentum, Peritoneum und/oder inneren Organen isoliert werden.

12. Verfahren nach einem der Ansprüche 8 - 11, **dadurch gekennzeichnet, daß** zur Auf- und/oder Einbringung der Zellbestandteile auf und/oder in die Oberfläche der Werkstoffe eine chemische Immobilisierung, Photoimmobilisierung, Adhäsion, Trocknung oder eine Kombination davon durchgeführt wird.

## Claims

1. Hemocompatible surfaces, **characterized in that** they contain as materials artificial and/or natural organic and/or inorganic compounds and/or mixtures thereof and/or materials having contact with blood and/or other body fluids in invasive operations and/or animal organs and/or organ parts, and oligosaccharide, polysaccharide and/or lipid portions of the glycoproteins, glycolipids and/or proteoglycans from the outer layer of blood cells and/or mesothelial cells are applied and/or incorporated onto and/or into the surfaces of said materials.

2. The hemocompatible surfaces according to claim 1, **characterized in that** they are non-thrombogenic and/or non-immunogenic.

3. The hemocompatible surfaces according to one of claims 1 or 2, containing glycophorins on and/or in the surfaces of the materials.

4. The hemocompatible surfaces according to one of claims 1 - 3, containing glycosphingolipids on and/or in the surfaces of the materials.

5. The hemocompatible surfaces according to one of claims 1 - 4, containing on and/or in the surfaces of the materials as the oligosaccharide and/or polysaccharide portions of the proteoglycans hyaluronic acids, chondroitin sulfates, dermatan sulfates, heparan sulfates, keratan sulfates or mixtures thereof.

6. The hemocompatible surfaces according to one of claims 1 - 5, containing on and/or in the surfaces of the materials heparan sulfate of the erythrocyte plasma membrane of animal and/or human origin.

7. The hemocompatible surfaces according to one of claims 1 - 6, containing as the materials high-molecular organic compounds and/or metals, metal oxides, alloys, ceramics, glasses, minerals and/or mixtures of the materials mentioned before.

8. A process for making hemocompatible surfaces, **characterized in that**
a) glycophorins and/or oligosaccharide, polysaccharide and/or lipid portions of the glycoproteins, glycolipids and/or proteoglycans are isolated from the outer layer of blood cells and/or mesothelial cells, and
b) said cell constituents are applied and/or incorporated onto and/or into the surfaces of materials of artificial and/or natural organic and/or inorganic compounds and/or mixtures thereof and/or materials having contact with blood and/or other body fluids in invasive operations and/or animal organs and/or organ parts by physical or chemical bonding.

9. The process according to claim 8, **characterized in that** the constituents from the outer layer of blood cells are isolated from whole blood and/or from cell fractions obtained therefrom of human or animal origin.

10. The process according to one of claims 8 or 9, **characterized in that** cell constituents are isolated from erythrocytes, leucocytes and/or thrombocytes and/or mixtures thereof.

11. The process according to one of claims 8 - 10, **characterized in that** constituents from the outer layer of mesothelial cells are isolated from omentum, peritoneum and/or inner organs.

12. The process according to one of claims 8 - 11, **characterized in that** a chemical immobilization, photoimmobilization, adhesion, drying process or a combination thereof is carried out for applying and/or incorporating the cell constituents onto and/or into the surfaces of the materials.

## Revendications

1. Surfaces hémocompatibles, **caractérisées en ce qu'**elles contiennent en tant que matériaux des composés inorganiques et/ou organiques naturels et/ou artificiels et/ou des mélanges de ceux-ci et/ou des matières qui entrent en contact avec le sang et/ou d'autres liquides biologiques lors d'interventions invasives et/ou des organes animaux et/ou des parties d'organes et des portions de lipides et/ou de polysaccharides, d'oligosaccharides des glycoprotéines, glycolipides et/ou des protéoglycanes de la couche externe des globules sanguins et/ou des cellules mésothéliales sont appliquées et/ou incorporées sur et/ou dans les surfaces desdits matériaux.

2. Surfaces hémocompatibles selon la revendication 1, **caractérisées en ce qu'**elles sont non thrombogènes et/ou non immunogènes,

3. Surfaces hémocompatibles selon l'une quelconque des revendications 1 ou 2, contenant des glycophorines sur et/ou dans les surfaces des matériaux.

4. Surfaces hémocompatibles selon l'une quelconque des revendications 1 à 3, contenant des glycosphingolipides sur et/ou dans les surfaces des matériaux.

5. Surfaces hémocompatibles selon l'une quelconque des revendications 1 à 4, contenant sur et/ou dans les surfaces des matériaux telles que des portions d'oligosaccharides et/ou de polysaccharides, d'acides hyaluroniques protéoglycanes, de chondroïtines sulfates, de dermatanes sulfates, d'héparanes sulfates, de kératanes sulfates ou des mélanges de ceux-ci.

6. Surfaces hémocompatibles selon l'une quelconque des revendications 1 à 5, contenant sur et/ou dans les surfaces des matériaux des héparanes sulfates de la membrane plasmique des hématies d'origine humaine et/ou animale.

7. Surfaces hémocompatibles selon l'une quelconque des revendications 1 à 6, contenant en tant que matériaux des composés organiques à haut poids moléculaire et/ou des métaux, des oxydes métalliques, des alliages, des céramiques, des verres, des minéraux et/ou des mélanges des matériaux mentionnés ci-dessus.

8. Procédé de fabrication de surfaces hémocompatibles, **caractérisé en ce que**
a) les glycophorines et/ou les portions de lipides et/ou de polysaccharides, d'oligosaccharides des glycoprotéines, glycolipides et/ou protéoglycanes sont isolées de la couche externe des globules sanguins et/ou des cellules mésothéliales et
b) lesdits constituants des cellules sont appliqués et/ou incorporés sur et/ou dans les surfaces des matériaux des composés inorganiques et/ou organiques naturels et/ou artificiels et/ou des mélanges de ceux-ci et/ou des matériaux qui entrent en contact avec le sang et/ou d'autres liquides biologiques lors d'interventions invasives et/ou des organes animaux et/ou des parties d'organes par liaison chimique ou physique.

9. Procédé selon la revendication 8, **caractérisé en ce que** les constituants de la couche externe des cellules sanguines sont isolés du sang complet et/ou des fractions de cellules obtenues d'origine animale ou humaine.

10. Procédé selon l'une quelconque des revendications 8 ou 9, **caractérisé en ce que** les constituants des cellules sont isolés des hématies, leucocytes et/ou thrombocytes et/ou mélanges de ceux-ci.

11. Procédé selon l'une quelconque des revendications 8 à 10, **caractérisé en ce que** les constituants de la couche externe des cellules mésothéliales sont isolés de l'épiploon, du péritoine et/ou organes internes.

12. Procédé selon l'une quelconque des revendications 8 à 11, **caractérisé en ce qu'**une immobilisation chimique, une photo-immobilisation, une adhérence, un séchage ou une combinaison de ceux-ci est effectué pour appliquer et/ou incorporer les constituants des cellules sur et/ou dans les surfaces des matériaux.
